# EUROPEAN PATENT APPLICATION

(11) **EP 1 897 494 A1**
(43) Date of publication of application: **12.03.2008**
(21) Application number: 07115106.2
(22) Date of filing: 28.08.2007
(51) Int. Cl.: A61B 5/20

(54) **Sensor for measuring bladder volume and system and method of managing bladder using the same**

(30) Priority: 31.08.2006 KR 20060083325
(71) Applicant: Gwangju Institute of Science and Technology, Buk-gu, Gwangju-si (KR)
(72) Inventor: Lee, Sun Kyu, Gwangju (KR); Kim, Kang Wook, Gwangsan-gu, Gwangju (KR)
(74) Representative: Capasso, Olga

(57) **Abstract**

The invention relates to a sensor for measuring bladder volume that includes an RFID chip (particularly, a passive RFID chip) to generate driving power using an electric wave generated by a reader, and to transmit information on the measured bladder volume to the reader using an elastic wave or an electromagnetic wave, and to a system and method of managing a bladder using the same.

A sensor for measuring bladder volume according to an embodiment of the invention includes an antenna (252) that receives an electric wave, a transducer (250) generates a predetermined wave and measures the bladder volume indicating the size of a bladder using the predetermined wave, and an RFID chip (254) that generates and supplies power using the electric wave received by the antenna (252), generates information on the bladder volume from the measured bladder volume, and transmits the generated information to the outside through the antenna (252). The sensor for measuring bladder volume is attached to the skin in an abdominal region of a user.

According to the embodiment of the invention, since the sensor for measuring bladder volume does not use a battery, it is reduced in size, thereby improving mobility. Therefore, an adverse affect when the sensor is inserted into the body through a surgical operation can be prevented.

## Description

### BACKGROUND OF THE INVENTION

### 1. Technical Field

The present invention relates to a sensor for measuring bladder volume, and a system and method of managing a bladder using the same. In particular, the present invention relates to a sensor for measuring bladder volume that includes a passive RFID (Radio Frequency IDentification) chip to generate driving power using an electric wave generated by a reader, and to transmit information on the bladder volume measured by an elastic wave or an electromagnetic wave to the reader, and to a system and method of managing a bladder using the same.

### 2. Related Art

Generally, the urinary bladder (hereinafter, simply referred to as "bladder") is a sac-like internal organ that is in the abdomen. The bladder stores urine from the kidney and eliminates it from the body through the urethra.

If healthy people receive, through the autonomic nervous system, a stimulus indicating that the bladder is full, they can appropriately respond to the stimulus. However, since people with disorders, such as dementia or paraplegia, in which the autonomic nervous system does not normally function, may not be able to receive the stimulus, which signals the bladder is full. For this reason, people with disorders or people who care for people with disorders (hereinafter, simply referred to as "caretaker") need to carry a storing container, such as a bag or a disposable absorbent article, or spare clothes, which causes an uncomfortable feeling of embarrassment for people with disorders. Further, if a caretaker is unavailable to take care of a person with a disorder, and the person with the disorder may be unable to care for himself/herself, unsanitary conditions may occur, such as urine stained clothes. In addition, if the bladder is kept full, urine flows back to the kidney and enters the bloodstream, which causes uremia and damages the kidney.

Accordingly, people with disorders, such as dementia or paraplegia, need an apparatus to inform the user that the bladder is full. Particularly, in recent years, since people with disorders, such as dysfunctional autonomic nervous system, are increased due to environmental pollution caused by industrialization, the need for the above-described apparatus will be in demand.

To this end, various apparatuses have been suggested. For example, as a medical instrument approved by the Korean Food & Drug Administration, there is known a urodynamics measurement system that measures the bladder volume and pressure. A description will now be given for the known apparatus or method that measures the bladder volume.

In the Ultrasonics Symposium, IEEE 1986, pp. 953-956, "Bladder Distension Sensor for the Handicapped" (hereinafter, referred to as "Related Document 1") by J. Companion, S. Heyman, T. Blalock, A. Cavalier, B. Mineo, F. Klien, and L. Fox, there is suggested an apparatus that measures the bladder volume using an ultrasonic wave. That is, according to Related Document 1, an ultrasonic sensor (or acoustic transducer) is attached to the skin of the abdomen close to the bladder of the subject. The ultrasonic sensor generates an ultrasonic wave, outputs the generated ultrasonic wave to the bladder, and receives the ultrasonic wave reflected from the bladder. Then, the waveform of the ultrasonic wave is analyzed, thereby grasping the bladder volume.

According to Related Document 1, power for driving the ultrasonic sensor is either obtained through a battery or supplied from a power supply that is connected to the ultrasonic sensor through a cable. When the ultrasonic sensor is supplied with power through the battery, it can only be used for a predetermined period due to the limitation of battery capacity. Accordingly, after the predetermined period is expired, it is inconvenient for the user to change the battery, and thus additional cost is required. Further, when the ultrasonic sensor is supplied with power through the power supply, if the power supply is too far from the ultrasonic sensor, the ultrasonic sensor cannot be sufficiently supplied with power due to the limited length of the cable. In addition, in order for the ultrasonic sensor to be sufficiently supplied with power, it is inconvenient for the user to constantly carry a cable.

Further, since the apparatus suggested in Related Document 1 is supplied with power as described above, the size of the ultrasonic sensor is large, and thus it is inconvenient for the user to carry the ultrasonic sensor.

In U.S. Patent Publication No. 2005/0165317 (July 28, 2005), entitled "Medical Devices" (hereinafter, referred to as "Related Document 2"), there is suggested an apparatus that is provided with a sensor in the body to more accurately measure the bladder volume. However, like Related Document 2, if the sensor is provided in the body, the body may adversely react (for example, a pain may occur). In this case, a predetermined part of the body, into which the sensor is inserted, needs to be protected from an external impact. Further, a surgical operation is needed to implant the sensor in the body, which results in time and cost. In addition, since the sensor needs to be made with a material that is harmless to people (that is, packaging using an expensive material having affinity to the organs of the body is needed), additional cost is required.

In U.S. Patent Publication No. 2004/0100376 (May 27, 2004) entitled "Healthcare Monitoring System" (hereinafter, referred to as "Related Document 3"), there is suggested an apparatus that, when a sensor attached to the body measures the bladder volume, transmits the measured bladder volume to a reader using a UWB (Ultra Wide Band) . However, since the UWB, as the communication means in Related Document 3, has an ultra wideband characteristic over several GHz, it inevitably coexists with a radio communication system, such as a CDMA or a GPS. That is, the UWB interferes with an apparatus using other radio communication means. Further, the UWB uses a wide band during communication, and the structure of the sensor is complicated, which leads to high cost. In addition, since the UWB needs high instantaneous pulse power, an electromagnetic problem occurs, but the antenna designed to be suitable for the ultra wideband characteristic becomes complicated. Furthermore, since the UWB has a high signal to noise ratio, the band and transmission power used need to be limited for frequency sharing.

In addition, the apparatus in Related Document 3 uses a small battery, such as a clock battery or a thin-film battery, as the power supply. Accordingly, the same problem as that in Related Document 1 occurs.

### SUMMARY OF THE INVENTION

An object of the invention is to provide a sensor for measuring bladder volume that, after the sensor acquires information on the bladder volume, transmits the acquired information on the bladder volume to a reader using an RFID chip (particularly, a passive RFID chip) without being affected by other peripheral radio communication apparatuses, and simplifies a structure, thereby reducing costs, and a system and method of managing a bladder using the same.

Another object of the invention is to provide a sensor for measuring bladder volume that is attached to skin (particularly, skin in an abdominal region), thereby saving time and cost of a surgical operation and eliminating risk when it is implanted in the body, and to a system and method of managing a bladder.

Still another object of the invention is to provide a sensor for measuring bladder volume that is supplied with driving power from a thermo-electric generating element, which uses an electric wave from a reader or heat from a body, thereby avoiding a problem occurring when power is supplied from a battery or a power supply connected thereto through a cable, and to a system and method of managing a bladder.

Yet still another object of the invention is to provide a sensor for measuring bladder volume that is manufactured compact and portable, thereby improving mobility, and to a system and method of managing a bladder.

Yet still another object of the invention is to provide a sensor for measuring bladder volume that includes a portable reader, thereby allowing a caretaker to immediately check that a bladder of a person with disorder is full, and reducing the amount of time a caretaker is needed, and a system and method of managing a bladder.

According to an aspect of the invention, a sensor for measuring bladder volume includes an antenna that receives an electric wave, a transducer that generates a predetermined wave and measures the bladder volume indicating the size of the bladder using the predetermined wave, and an RFID chip that generates and supplies power using the electric wave received by the antenna, generates information on the bladder volume from the measured bladder volume, and transmits the generated information to the outside through the antenna. The sensor for measuring bladder volume is attached to the skin in an abdominal region of a user.

The predetermined wave generated by the transducer may be an elastic wave or an electromagnetic wave.

The RFID chip may include a biological thermo-electric generating element that generates power using the body temperature.

The RFID chip may have one of information on the user, information for identifying the user, and a unique ID.

According to another aspect of the invention, there is provided a system for managing a bladder that uses a sensor for measuring bladder volume. The system includes a bladder RFID reader that transmits an electric wave cyclically or when necessary, if receiving information on the bladder volume indicating the size of a bladder of a user, analyzing the information, and informs the user or a caretaker of the analysis result, and a sensor for measuring bladder volume that generates power using an electromagnetic field by the electric wave, generates the information on the bladder volume using a predetermined wave, and transmits the generated information to the bladder RFID reader.

The bladder RFID reader may include a device, such as an alarm device, a sound device, a display device, or a light emitting diode device, which allows the user or the caretaker to recognize a bladder condition of the user.

The bladder RFID reader may be a mobile apparatus that is carried by the user or the caretaker, or a fixed apparatus that is attached to a wall, a pillar, or a ceiling.

The system for managing a bladder may further include a bladder management server that receives the information on the bladder volume of the user from the bladder RFID reader, manages a history of a bladder condition of the user using the information on the bladder volume, predicts the bladder condition of the user in an emergency, and informs the user or the caretaker of the prediction result, a bladder management database that stores information received and managed by the bladder management server in connection with the bladder management server, and a caretaker terminal, which is used by the caretaker to connect to the bladder RFID reader or the bladder management server, and through which the caretaker is informed of the bladder condition of the user from the bladder RFID reader or the bladder management server.

According to still another aspect of the invention, there is provided a method of operating a system that uses a sensor for measuring bladder volume. The method includes the steps of (a) causing a bladder RFID reader to generate an electric wave cyclically or whenever demanded by a specific person, (b) causing a sensor for measuring bladder volume to receive the electric wave to generate power, to generate an elastic wave or an electromagnetic wave, to output the generated elastic wave or electromagnetic wave toward a bladder of a user, (c) causing the sensor for measuring bladder volume to receive the elastic wave or the electromagnetic wave reflected from the bladder and to generate information on the bladder volume of the user from the reflected elastic wave or electromagnetic wave, (d) causing the sensor for measuring bladder volume to transmit a signal having the information on the bladder volume to the bladder RFID reader, and (e) causing the bladder RFID reader to inform the user or a caretaker of a bladder condition of the user.

The step (b) may include a substep of (a2) causing the sensor for measuring bladder volume to transmit the stored information on the bladder volume to the bladder RFID reader.

### BRIEF DESCRIPTION OF THE DRAWINGS

FIG. 1 is a conceptual view schematically illustrating a system of managing a bladder according to an exemplary embodiment of the invention;
FIG. 2A is a block diagram schematically showing the internal configuration of a sensor for measuring bladder volume in the system for managing a bladder according to the exemplary embodiment of the invention;
FIG. 2B is a detail view showing an example of the sensor for measuring bladder volume in the system for managing a bladder according to the exemplary embodiment of the invention;
FIG. 3A is a diagram showing a first example of a bladder RFID reader in the system for managing a bladder according to the exemplary embodiment of the invention;
FIG. 3B is a diagram showing a second example of a bladder RFID reader in the system for managing a bladder according to the exemplary embodiment of the invention;
FIG. 3C is a diagram showing a third example of a bladder RFID reader in the system for managing a bladder according to the exemplary embodiment of the invention; and
FIG. 4 is a flowchart illustrating a method of operating a system according to an exemplary embodiment of the invention.

### DESCRIPTION OF EXEMPLARY EMBODIMENTS

Hereinafter, exemplary embodiments of the invention will be described with reference to the accompanying drawings. It should be noted that, in the drawings, the same parts are represented by the same reference numerals. In the embodiments of the invention, detailed description of known structures and functions incorporated herein will be omitted when it may make the subject matter of the present invention unclear. Although the invention has been described in connection with the exemplary embodiments of the invention, it will be apparent to those skilled in the art that various modifications and changes may be made thereto without departing from the scope and spirit of the invention. Therefore, it should be understood that the above embodiments are not limitative, but illustrative in all aspects.

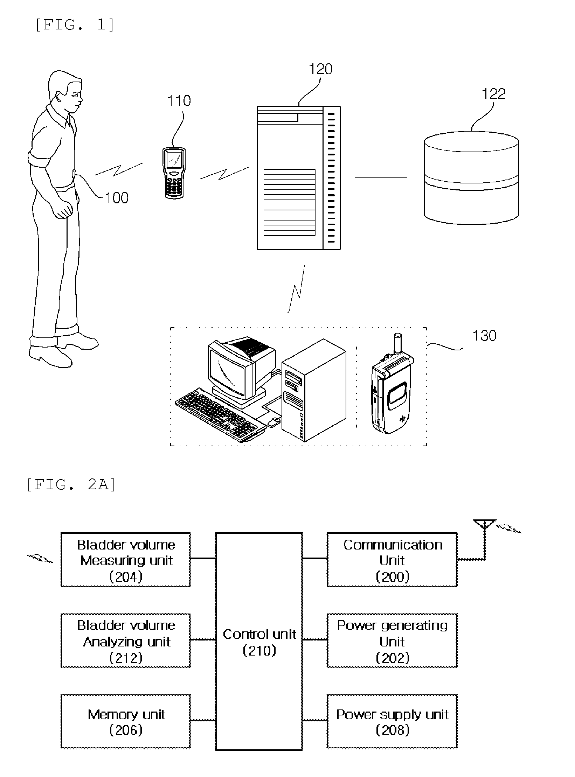

FIG. 1 is a conceptual view schematically illustrating a system of managing a bladder according to an exemplary embodiment of the invention. Referring to FIG. 1, a system for managing a bladder (hereinafter, also referred to as "bladder management system") according to an exemplary embodiment of the invention includes a sensor 100 for measuring bladder volume (hereinafter, also referred to as "bladder volume measuring sensor 100"), a bladder RFID reader 110, a bladder management server 120, a bladder management database 122, and a caretaker terminal 130.

In the bladder management system according to the exemplary embodiment of the invention, when the bladder RFID reader 110 requests the bladder volume measuring sensor 100 attached to the skin of a person with a disorder (hereinafter, referred to as "user") for information on the bladder volume of the user, the bladder volume measuring sensor 100 transmits the information on the bladder volume to the bladder RFID reader 110. Then, on the basis of the transmitted information, the bladder RFID reader 110 informs the user or a caretaker that the bladder condition is abnormal (for example, the bladder is full) . A description will now be given for a method that allows the bladder RFID reader 110 to acquire information on the bladder volume from the bladder volume measuring sensor 100.

First, if the bladder RFID reader 110 transmits a signal having a message to request the information on the bladder volume, the bladder volume measuring sensor 100 receives the corresponding signal. Then, the bladder volume measuring sensor 100 transmits the information on the measured bladder volume to the RFID reader 110. At this time, if the information on the measured bladder volume does not exist, the bladder volume measuring sensor 100 acquires the information on the volume from the bladder using an elastic wave (for example, ultrasonic wave) or an electromagnetic wave, and transmits the acquired information to the bladder RFID reader 110. The method that allows the bladder volume measuring sensor 100 to acquire the information on the volume from the bladder will be described below in detail.

Preferably, the bladder volume measuring sensor 100 transmits user information to the bladder RFID reader 110, together with the information on the bladder volume. This is because, if the bladder volume measuring sensor 100 transmits the user information, it is possible to identify from whom the information on the bladder volume is. In addition, the bladder volume measuring sensor 100 may transmit information for identifying a user or a unique ID of an RFID chip, instead of the user information.

The bladder management system according to the exemplary embodiment of the invention can be provided in medical institutes, such as hospitals, and used to care for people with disorders or old people.

The bladder volume measuring sensor 100 according to the exemplary embodiment of the invention measures the bladder volume of the user. To this end, the bladder volume measuring sensor 100 includes a transducer. That is, the bladder volume measuring sensor 100 generates an elastic wave or an electromagnetic wave using the transducer. Then, the bladder volume measuring sensor 100 outputs the elastic wave or the electromagnetic wave toward the bladder, and receives the reflected wave from the bladder. Accordingly, the bladder volume measuring sensor 100 can acquire the information on the bladder volume on the basis of the received wave.

When the bladder RFID reader 110 requests the information on the bladder volume, the bladder volume measuring sensor 100 transmits the information on the measured bladder volume to the bladder RFID reader 110. To this end, the bladder volume measuring sensor 100 includes an antenna. Alternatively, the bladder volume measuring sensor 100 may transmit the information on the bladder volume to the bladder RFID reader 110 immediately after measurement.

The bladder volume measuring sensor 100 according to the exemplary embodiment of the invention generates driving power using an electric wave from the bladder RFID reader 110 to be then autogenous. As described above, a known sensor that measures the bladder volume is connected to a battery or a power supply in a wired manner and is supplied with driving power. If power is supplied from the battery, a battery needs to be replaced when necessary, and the sensor is attached and detached to and from the abdomen of the user. Further, if power is supplied from the power supply, mobility of the user, to whom the sensor is attached, is limited. In contrast, since the bladder volume measuring sensor 100 according to the embodiment of the invention generates power, the above-described problems can be resolved.

The bladder volume measuring sensor 100 according to the embodiment of the invention includes an RFID chip in order to achieve the above-described object. Particularly, the bladder volume measuring sensor 100 includes a passive RFID chip. Usually, an RFID reader transmits a predetermined electric wave to the RFID chip so as to activate the RFID chip, supplies power, and recognizes data stored in the RFID chip through encoding and decoding. That is, if the bladder RFID reader 110 outputs a signal having a message to request the information on the bladder volume cyclically (or when necessary), the bladder volume measuring sensor 100 receives the corresponding signal through the antenna. At this time, the bladder RFID reader 110 excites the bladder volume measuring sensor 100 using inductive coupling or electromagnetic capturing. Then, the bladder volume measuring sensor 100 can generate and ensure power from an electromagnetic field (or RF field) incident on the antenna.

Preferably, the bladder volume measuring sensor 100 is compact such that the user can conveniently carry it with him/her. Since the passive RFID chip is not provided with a battery or a power supply, it can be compact. Therefore, if the bladder volume measuring sensor 100 includes the passive RFID chip, the sensor can be reduced in size.

Meanwhile, the passive RFID chip has a considerably short recognizable range, for example, approximately 1 m. Although the UHF (Ultra High Frequency) of 900 MHz is recently introduced and applied to the RFID, the recognizable range is approximately 5 m. That is, in order to avoid interference with other radio communication devices (for example, CDMA or GPS terminal), the amount of power by an electric wave from the bladder RFID reader 110 is considerably small. For this reason, if the bladder RFID reader 110 is separated from the passive RFID chip of the bladder volume measuring sensor 100 by 5 m or more, the recognition rate is remarkably decreased and practicality is deteriorated. Accordingly, the bladder RFID reader 110 is located close to the bladder volume measuring sensor 100. Therefore, it is preferable that the user, to whom the bladder volume measuring sensor 100 is attached, carry the bladder RFID reader 110 with him/her. Alternatively, the bladder RFID reader 110 may be fixed to a wall or a ceiling. In this case, the bladder RFID reader 110 is preferably provided in a short distance.

The RFID chip in the bladder volume measuring sensor 100 may be an active type or a semi-passive type. If the RFID chip is the active type, the recognizable range extends to tens of meters. However, since the active RFID chip requires high manufacturing cost and uses a battery, the available period of time is limited. The semi-passive RFID chip operates using a battery, but power can be generated using the electric wave from the bladder RFID reader 110.

To ensure sufficient power, the bladder volume measuring sensor 100 preferably further includes a biological thermo-electric generating element that generates power using heat from the body (that is, body temperature). The biological thermo-electric generating element has the following configuration, for example.

Generally, as an electrical method that is applied to temperature measurement, a thermo couple is most widely used. The thermo couple is a temperature sensor that has two different materials, which come into thermal and electrical contact with each other. In the thermo couple, a potential difference is generated at the contact of the two materials according to a change in temperature. This phenomenon is called a thermo-electric effect, and the potential difference is called a thermo-electromotive force. The thermo-electric effect is classified into a Seebeck effect that obtains an electromotive force using a difference in temperature between both ends of the thermo couple, and a Peltier effect that obtains an electromotive force by energy discontinuity between different materials.

Accordingly, if the device is formed to include the thermo couple, the biological thermo-electric generating element according to the embodiment of the invention can be manufactured. At this time, the thermo is preferably formed of a metal having a high thermo-electromotive force, good heat resistance and corrosion resistance, high mechanical strength, stable thermo-electromotive force for a long time, small damage and abrasion, the same characteristic in the same kind of thermo couple, compatibility, and low electrical resistance and temperature coefficient when high-temperature measurement.

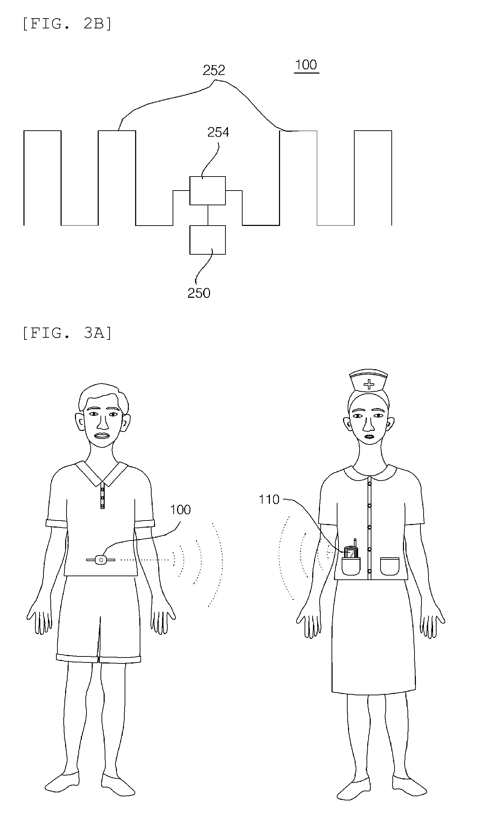

The internal configuration of the bladder volume measuring sensor 100 will be described below with reference to FIGS. 2A and 2B.

The RFID chip is provided as an IC (Integrated Circuit) chip. However, the RFID chip is not limited thereto. For example, The RFID chip may be provided as an IC tag or an IC card.

The bladder RFID reader 110 generates an electric wave cyclically and outputs the generated electric wave to the bladder volume measuring sensor 100. Here, the electric wave output to the bladder volume measuring sensor 100 has a message to request the information on the bladder volume. Then, the bladder volume measuring sensor 100 receives the electric wave, generates power, analyzes the message, and performs an appropriate action according to the analysis result.

If a signal having the information on the bladder volume is transmitted from the bladder volume measuring sensor 100, the bladder RFID reader 110 receives the corresponding signal. Then, the bladder RFID reader 110 analyzes the received information and determines the bladder condition of the user. At this time, if it is determined that the bladder condition of the user is abnormal (that is, the bladder of the user is full), the bladder RFID reader 110 informs the user or the caretaker that the bladder condition of the user is abnormal. To this end, the bladder RFID reader 110 includes an alarm device, a sound device, a display device, or a light-emitting diode device, such that the user or the caretaker recognizes the bladder condition.

The bladder RFID reader 110 includes a storage device, such as a flash memory, to store information transmitted from the bladder volume measuring sensor 100. In this case, the bladder RFID reader 110 stores a history on the bladder condition of the user.

The bladder RFID reader 110 is a mobile apparatus that is carried by the user or the caretaker. Alternatively, the bladder RFID reader 110 may be a fixed apparatus that is fixed to a wall or a ceiling. When the bladder RFID reader 110 is a fixed apparatus, the recognizable range is increased compared with the mobile apparatus. However, if the bladder RFID reader 110 is the fixed apparatus, it is impossible to identity from whom the information on the bladder volume is. Accordingly, when the bladder RFID reader 110 is the fixed apparatus, the bladder RFID reader 110 preferably acquires user information from the bladder volume measuring sensor 100, together with the information on the bladder volume. Then, the bladder RFID reader 110 can identify from whom the information on the bladder volume is, and take appropriate action.

The bladder RFID reader 110 according to the embodiment of the invention may be implemented along with a device for advanced system operation, such as an anti-collision system that can simultaneously read a plurality of bladder volume measuring sensors 100, a dense reader communication mode that is used in an environment, in which various readers are densely installed.

Specific examples of the bladder RFID reader 110 will be described below with reference to FIGS. 3A to 3C.

The bladder management server 120 receives the information on the bladder volume of the user generated by the bladder RFID reader 110 and stores the received information in the bladder management database 122. Then, the bladder management server 120 can generate and manage a history on the bladder condition of the user on the basis of the information stored in the bladder management database 122. Further, the bladder management server 120 predicts the bladder condition of the user on the basis of the history stored in the bladder management database 122. In this case, the bladder management server 120 may further include a bladder condition prediction system. The bladder condition prediction system predicts the bladder condition of the user on the basis of time information, information on the habit and physical constitution of the user (for example, information on the habit of the user who gets a drink at a predetermined time), and the like, in addition to the history on the bladder condition of the user. Then, in an emergency (for example, the bladder RFID reader 110 does not operate), the user or the caretaker can be informed of the prediction result by the bladder management server 120 and then take appropriate action.

When being informed from the bladder RFID reader 110 that the bladder condition of the user is abnormal, the bladder management server 120 informs the caretaker terminal 130 of the abnormality. To this end, the bladder management server 120 includes a device that is connected to various wired or wireless communication networks, such as a CDMA network, a GPS network, and Internet network. Then, even if the caretaker is at a place other than where the user is, for example, far away, the caretaker can get in touch with another person (for example, a nurse or a family) who is located close to the user, such that appropriate action can be taken. Alternatively, the bladder RFID reader 110 may directly inform the caretaker terminal 130 of information on the abnormality. At this time, the bladder RFID reader 110 also includes a device that is connected to the wired and wireless communication networks.

The bladder management server 120 can receive the information on the bladder volume from the bladder RFID reader 110 and analyze the information on the bladder volume.

The bladder volume measuring sensor 100 according to the embodiment of the invention is provided for people with disorders or old people who are in the hospital. Accordingly, the bladder management server 120 is provided in a hospital where many people with disorders or old people may be residing. However, a place where the bladder management server 120 is provided is not limited thereto.

The bladder management server 120 processes a request from the bladder RFID reader 110 or the caretaker terminal 130 or provides a service thereto through a wired or wireless communication network in a distributed client/server system, which operates one or more application programs such that the client can obtain a desired result through collaboration between the client, which requests a service, and a server, which processes the request from the client. The bladder management server 120 may include an application program for providing various services, in addition to a general server program.

The bladder management database 122 provides a database that records or stores information from the bladder management server 120 or provides information stored therein. The bladder management database 122 according to the embodiment of the invention stores the information on the bladder volume of the user, the history on the bladder condition of the user, the time information, and the information on the habit and physical constitution of the user. However, the information stored in the bladder management database 122 is not limited thereto.

The caretaker terminal 130 is a terminal that can be used by the caretaker who cares for the user. The caretaker terminal 130 may be a general cellular phone, but the invention is not limited thereto. For example, the caretaker terminal 130 may be a PC (Personal Computer). Moreover, the caretaker who cares for the user refers to a medical attendant, such as a nurse, or a family member.

Next, the internal configuration of the bladder volume measuring sensor 100 will be described in detail.

FIG. 2A is a block diagram schematically showing the internal configuration of a bladder volume measuring sensor in the bladder management system according to the exemplary embodiment of the invention. Referring to FIG. 2A, a bladder volume measuring sensor 100 according to an exemplary embodiment of the invention includes a communication unit 200, power generating unit 202, a bladder volume measuring unit 204, a memory unit 206, a power supply unit 208, a control unit 210, and a bladder volume analyzing unit 212.

The communication unit 200 receives the electric wave that is cyclically transmitted from the bladder RFID reader 110 . To this end, the communication unit 200 includes an antenna.

Further, when the bladder volume measuring unit 204 acquires the information on the bladder volume of the user, the communication unit 200 transmits the information on the bladder volume to the bladder RFID reader 110. At this time, the communication unit 200 acquires the user information stored in the memory unit 206 through the control unit 210 and transmits the user information together with the information on the bladder volume.

The power generating unit 202 generates power using the electromagnetic field (or RF field) incident on the antenna of the communication unit 200 when the communication unit 200 receives the electric wave. The power generating unit 202 can generate power using heat from the body. In this case, the power generating unit 202 includes a biological thermo-electric generating element.

The bladder volume measuring unit 204 generates the elastic wave or the electromagnetic wave cyclically or when necessary. Then, the bladder volume measuring unit 204 receives the wave that returns after interaction with the bladder. Accordingly, the bladder volume analyzing unit 212 acquires the information on the bladder volume from the received wave.

The bladder volume analyzing unit 212 generates the information on the bladder volume on the basis of the wave received by the bladder volume measuring unit 204.

The memory unit 206 stores the information on the bladder volume of the user acquired by the bladder volume measuring unit 204, the user information, and the unique information for identifying the user.

The power supply unit 208 is supplied with power from the power generating unit 202 and supplies the power to the bladder volume measuring sensor 100 to smoothly operate.

The control unit 210 controls the overall operation of the bladder volume measuring sensor 100.

The bladder volume measuring sensor 100 may be implemented as follows.

FIG. 2B is a detail view showing an example of the bladder volume measuring sensor in the bladder management system according to the exemplary embodiment of the invention. Referring to FIG. 2B, the bladder volume measuring sensor 100 includes a transducer 250, an antenna 252, and an RFID chip 254.

The functions of the units 250 to 254 have been already described with reference to FIG. 1, and thus the detailed descriptions thereof will be omitted.

The transducer 250 generates the elastic wave or the electromagnetic wave in order to measure the bladder volume. The transducer 250 can generate the elastic wave or the electromagnetic wave using a wideband pulse or at least one narrowband frequency. The bladder volume measuring unit 204 shown in FIG. 2A corresponds to the transducer 250 shown in FIG. 2B.

The RFID chip 254 generates power using the electromagnetic field generated from the bladder RFID reader 110 and then incident on the antenna 252. Then, the RFID chip 254 is driven by the generated power. In addition, the RFID chip 254 supplies the generated power to the transducer 250 and so as to allow the transducer 250 to normally operate. Since the RFID chip 254 has a unique ID, even if a plurality of bladder volume measuring sensors 100 are used, the bladder RFID reader 110 can identify the bladder condition of each user.

The communication unit 200, the power generating unit 202, the memory unit 206, the power supply unit 208, the control unit 210, and the bladder volume analyzing unit 212, shown in FIG. 2A, are incorporated into the RFID chip 254 and the antenna 252 shown in FIG. 2B. The antenna 252 is provided on both sides of the RFID chip 254 in order to improve the reception rate of the electric wave, but the antenna 252 may be provided on one side.

Next, specific examples of the bladder RFID reader 110 will be described.

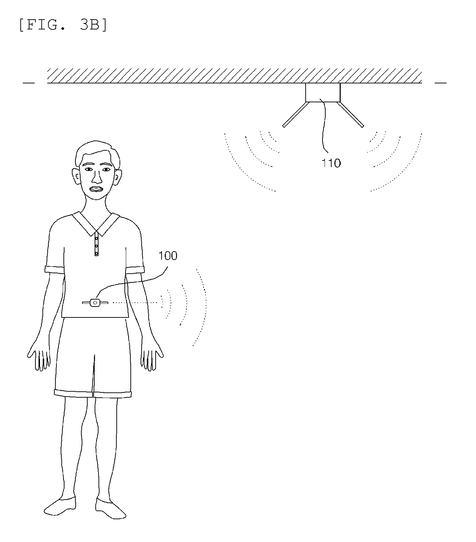

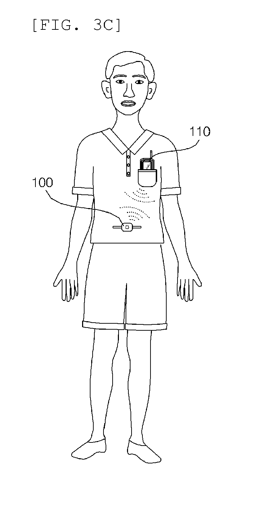

FIG. 3A is a diagram showing a first example of a bladder RFID reader in the bladder management system according to the exemplary embodiment of the invention. FIG. 3B is a diagram showing a second example of a bladder RFID reader in the bladder management system according to the exemplary embodiment of the invention. FIG. 3C is a diagram showing a third example of a bladder RFID reader in the bladder management system according to the exemplary embodiment of the invention.

Referring to FIG. 3A, the bladder volume measuring sensor 100 is attached to the skin in an abdominal region near the bladder of the user, like a sticker. The bladder RFID reader 110 is carried by a nurse (caretaker). Then, the nurse operates the bladder RFID reader 110 cyclically or when necessary and is able to read the bladder condition of the user on the basis of the information acquired by the bladder volume measuring sensor 100.

Referring to FIG. 3B, the bladder RFID reader 110 is a fixed apparatus that is fixed to a wall, a pillar, or a ceiling of a hospital or a home. The bladder RFID reader 110 acquires information from the bladder volume measuring sensor 100 cyclically or when demanded by a specific person, using an internal program. Then, on the basis of the acquired information, the bladder RFID reader 110 informs the caretaker or the user of the bladder condition of the user. To this end, the bladder RFID reader 110 includes an alarm device, a sound device, or a display device. However, the bladder RFID reader 110 is not limited to an RFID reader having such a device. For example, the bladder RFID reader 110 may include a device that is connected to various wired and wireless networks, such as Internet network and Intranet network, and may inform the caretaker or the user of the bladder condition.

The bladder RFID reader 110 can acquire information from one or more bladder volume measuring sensors 100. Further, the bladder RFID reader 110 is connected to various wired and wireless networks to be then controlled by the bladder management server 120, the caretaker terminal 130, or a user terminal. In this case, the bladder volume measuring sensor 100 is attached as shown in FIG. 3A.

Referring to FIG. 3C, the bladder RFID reader 110 is carried by the user. Then, the user operates the bladder RFID reader 110 cyclically or when necessary. If the bladder RFID reader 110 acquires the information on the bladder volume of the user, the user is informed of the corresponding information, such that he/she takes an appropriate action. Further, the bladder RFID reader 110 communicates with the bladder RFID reader 110 shown in FIG. 3A or 3B, such that the caretaker checks the information on the bladder volume.

The bladder RFID reader 110 can acquire specific information from other sensors that are attached to the user's body.

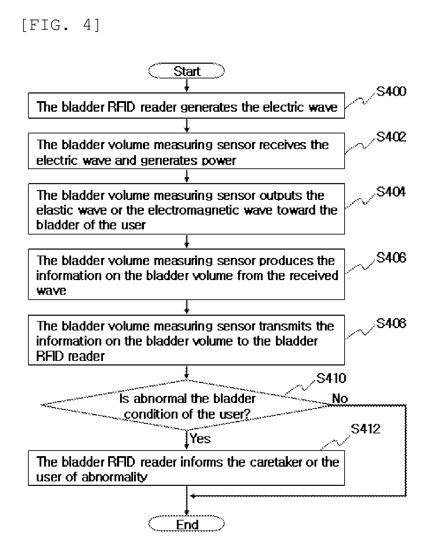

Next, a method of operating the bladder management system having the above-described configuration will be described. FIG. 4 is a flowchart illustrating a method of operating a system according to an exemplary embodiment of the invention.

Referring to FIG. 4, the bladder RFID reader 110 generates the electric wave cyclically or when demanded by a specific person. The electric wave has a message to request the information on the bladder volume of the user (Step S400).

The control unit 210 of the bladder volume measuring sensor receives the electric wave through the communication unit 200. At this time, the control unit 210 drives the power generating unit 202 to generate power using the electromagnetic field incident through the antenna of the communication unit 200 (Step S402). Further, in order to ensure sufficient driving power, the control unit 210 may operate the biological thermo-electric generating element in the power generating unit 202 when necessary. The generated power is stored in the power supply unit 208, and the power supply unit 208 uses the power to drive the bladder volume measuring sensor 100. Next, the control unit 210 controls the bladder volume measuring unit 204 to generate the elastic wave or the electromagnetic wave. Next, the control unit 210 outputs the wave toward the bladder of the user through the bladder volume measuring unit 204 (Step S404) .

If the elastic wave or the electromagnetic wave is reflected and returns after the interaction with the bladder, the bladder volume measuring unit 204 receives the reflected wave. The received signal includes the information on the bladder volume. Then, the bladder volume analyzing unit 212 acquires the information on the bladder volume from the received signal (Step S406). Next, the control unit 210 transmits the information on the bladder volume to the bladder RFID reader 110 through the communication unit 200 (Step 5408). At this time, the control unit 210 may read out the user information or the unique information for identifying the user from the memory unit 206 and control the communication unit 200 to transmit the user information or the unique information together with the information on the bladder volume.

According to the exemplary embodiment of the invention, the bladder volume measuring sensor 100 receives the electric wave from the bladder RFID reader 110 and then transmits the information on the bladder volume to the bladder RFID reader 110. Alternatively, the bladder volume measuring sensor 100 may acquire the information on the bladder volume cyclically or when necessary and immediately transmit the information on the bladder volume to the bladder RFID reader 110.

When receiving the information on the bladder volume, the bladder RFID reader 110 determines the bladder condition of the user (Step S410). If it is determined that the bladder condition of the user is abnormal, the bladder RFID reader 110 informs the caretaker or the user of abnormality (Step S412).

The bladder RFID reader 110 may transmit the determination result or the information on the bladder volume of the user to the bladder management server 120. Then, the bladder management server 120 can inform the caretaker terminal 130 or the user terminal of the determination result by the bladder RFID reader 110. Further, the bladder management server 120 can store a history on the bladder condition of the user on the basis of the information on the bladder volume. In this case, the bladder management server 120 can inform the caretaker or the user of the prediction result on the bladder condition of the user on the basis of the history in an emergency.

Although the present invention has been described in connection with the exemplary embodiments of the present invention, it will be apparent to those skilled in the art that various modifications and changes may be made thereto without departing from the scope and spirit of the invention. Therefore, it should be understood that the above embodiments are not limitative, but illustrative in all aspects. The scope of the present invention is defined by the appended claims rather than by the description preceding them, and all changes and modifications that fall within metes and bounds of the claims, or equivalents of such metes and bounds are therefore intended to be embraced by the claims.

As described above, according to the embodiments of the invention, since the bladder volume measuring sensor does not use a battery, the sensor can be reduced in size, thereby improving mobility. Further, an adverse effect when the sensor is implanted in the body through a surgical operation can be prevented.

According to the embodiments of the invention, the amount of work can be reduced for a caretaker who cares for an old person with dementia. This is because the amount of urine in the bladder of an old person with dementia is measured, and when the amount of urine in the bladder is large, the caretaker causes the old person with dementia to urinate in advance. In addition, if the amount of urine in the bladder is decreased for no particular reason, the caretaker can determine that urine has partially leaked, and can immediately change a diaper, thereby improving the sanitary condition.

According to the embodiments of the invention, the bladder volume measuring sensor can be used for people with paraplegia that cannot respond a stimulus, that is, control urination, thereby offering people with paraplegia convenience.

According to the embodiments of the invention, the bladder volume measuring sensor can be used to train a person with mental disability to urinate. This is because a nurse who carries the bladder RFID reader trains the person with mental disability by instructing the person with mental disability to urinate on the basis of the transmitted information. In addition, the bladder RFID reader can be carried by the person with mental disability, and the person with mental disability can perform the urination training by himself/herself.

## Claims

1. A sensor for measuring bladder volume, the sensor comprising:
an antenna (252) that receives an electric wave;
a transducer (250) that generates a predetermined wave and measures the bladder volume indicating the size of the bladder using the predetermined wave; and
an RFID chip (254) that generates and supplies power using the electric wave received by the antenna (252), generates information on the bladder volume from the measured bladder volume, and transmits the generated information to the outside through the antenna (252),
**characterized in that** the sensor for measuring bladder volume is attached to the skin in an abdominal region of a user.

2. The sensor of claim 1,
**characterized in that** the predetermined wave generated by the transducer (250) is an elastic wave or an electromagnetic wave.

3. The sensor of claim 1,
**characterized in that** the RFID chip (254) includes a biological thermo-electric generating element that generates power using the body temperature.

4. The sensor of any one of claims 1 to 3,
**characterized in that** the RFID chip (254) has one of information on the user, information for identifying the user, and a unique ID.

5. A system for managing a bladder that uses a sensor for measuring bladder volume, the system comprising:
a bladder RFID reader (110) that transmits an electric wave cyclically or when necessary, if receiving information on the bladder volume indicating the size of a bladder of a user, analyzing the information, and informs the user or a caretaker of the analysis result; and
a sensor (100) for measuring bladder volume that generates power using an electromagnetic field by the electric wave, generates the information on the bladder volume using a predetermined wave, and transmits the generated information to the bladder RFID reader (110).

6. The system of claim 5,
**characterized in that** the bladder RFID reader (110) adds, to the electric wave, a message to request the information on the bladder volume.

7. The system of claim 5,
**characterized in that** the bladder RFID reader (110) includes one of an alarm device, a sound device, a display device, and a light emitting diode device.

8. The system of claim 5,
**characterized in that** the bladder RFID reader (110) is a mobile apparatus that is carried by the user or the caretaker, or a fixed apparatus that is attached to a wall, a pillar, or a ceiling.

9. The system of claim 5,
**characterized in that** the sensor (100) for measuring bladder volume includes a biological thermo-electric generating element that generates power using body temperature.

10. The system of any one of claims 5 to 9,
**characterized in that** the sensor (100) for measuring bladder volume has one of information on the user, information for identifying the user, and a unique ID.

11. The system of claim 8,
**characterized in that** the sensor (100) for measuring bladder volume is supplied with power using a battery.

12. The system of claim 10, further comprising:
a bladder management server (120) that receives the information on the bladder volume of the user from the bladder RFID reader (110), manages a history of a bladder condition of the user using the information on the bladder volume, predicts the bladder condition of the user in an emergency, and informs the user or the caretaker of the prediction result; and
a bladder management database (122) that stores information received and managed by the bladder management server (120) in connection with the bladder management server (120).

13. The system of claim 12, further comprising:
a caretaker terminal (130), which is used by the caretaker to connect to the bladder RFID reader (110) or the bladder management server (120), and through which the caretaker is informed of the bladder condition of the user from the bladder RFID reader (110) or the bladder management server (120).

14. The system of claim 5,
**characterized in that** the sensor (100) for measuring bladder volume includes:
a communication unit (200) that includes an antenna to receive the electric wave and to transmit the information on the bladder volume to the outside;
a power generating unit (202) that generates power using the electromagnetic field incident through the communication unit (200) or heat generated from the body;
a bladder volume measuring unit (204) that generates an elastic wave or an electromagnetic wave as the predetermined wave cyclically or when necessary, and receives the reflected elastic wave or electromagnetic wave;
a bladder volume analyzing unit (212) that generates the information on the bladder volume on the basis of the wave received by the bladder volume measuring unit (204);
a power supply unit (208) that stores power generated by the power generating unit (202), and supplies power to the sensor (100) for measuring bladder volume to operate the sensor (100) for measuring bladder volume;
a control unit (210) that controls the overall operation of the sensor (100) for measuring bladder volume; and
a memory unit (206) that stores at least one of the information on the bladder volume, information on the user, information for identifying the user, and a unique ID.

15. A method of operating a system that uses a sensor for measuring bladder volume, the method comprising the steps of:
(a) causing a bladder RFID reader (110) to generate an electric wave cyclically or whenever demanded by a specific person;
(b) causing a sensor (100) for measuring bladder volume to receive the electric wave to generate power, to generate an elastic wave or an electromagnetic wave, to output the generated elastic wave or electromagnetic wave toward a bladder of a user;
(c) causing the sensor (100) for measuring bladder volume to receive the elastic wave or the electromagnetic wave reflected from the bladder and to generate information on the bladder volume of the user from the reflected elastic wave or electromagnetic wave;
(d) causing the sensor (100) for measuring bladder volume to transmit a signal having the information on the bladder volume to the bladder RFID reader (110); and
(e) causing the bladder RFID reader (110) to inform the user or a caretaker of a bladder condition of the user.

16. The method of claim 15,
**characterized in that** the step (a) includes a substep of:
(a1) causing the bladder RFID reader (110) to add, to the electric wave, a message to request information on the bladder condition of the user.

17. The method of claim 15,
**characterized in that** the step (b) includes a substep of:
(a2) causing the sensor (100) for measuring bladder volume to transmit the stored information on the bladder volume to the bladder RFID reader (110).

18. The method of claim 15,
**characterized in that** the step (d) includes a substep of:
(a3) causing the sensor (100) for measuring bladder volume to add, to the corresponding signal, one of information on the user, information for identifying the user, and a unique ID.

19. The method of claim 15,
**characterized in that** one of the steps (a) to (e) includes a substep of:
(a4) causing the sensor (100) for measuring bladder volume to drive a biological thermo-electric generating element that generates power using heat generated from the body.
